# EUROPEAN PATENT APPLICATION

(11) **EP 4 529 981 A1**
(43) Date of publication of application: **02.04.2025**
(21) Application number: 23213109.4
(22) Date of filing: 29.11.2023
(51) Int. Cl.: B01L 3/00, G01N 27/27, G01N 33/18, B82Y 15/00, B82Y 40/00, C12Q 1/00, C12Q 1/25, G01N 27/22, G01N 27/327

(54) **METHOD FOR MANUFACTURING A SENSING DEVICE FOR HEAVY METAL DETECTION IN A LIQUID AND SUCH A SENSING DEVICE**

(30) Priority: 29.09.2023 GR 20230100787
(71) Applicant: National Technical University of Athens (Ethniko Metsovio Politechnio), 10682 Athens (GR); Tsoukalas, Dimitrios, 16675 Glyfada, Attika (GR); Skotadis, Evangelos, 135 62 Agioi Anargiroi (GR)
(72) Inventor: Tsoukalas, Dimitrios, 16675 Glyfada (GR); Skotadis, Evangelos, 135 62 Agioi Anargiroi (GR); Chatzandroulis, Stavros, Athens (GR)
(74) Representative: Kouzelis, Dimitrios

(57) **Abstract**

The present invention refers to a sensing device (100) for heavy metal detection in a liquid, in particular water, comprising a substrate (30), a microfluidic channel (103) on the substrate (30), a MoS₂-based sensor (101), and a nanoparticle network sensor (102), which is functionalized with DNAzymes (10). The MoS₂-based sensor (101) and the nanoparticle network sensor (102) are integrated in the microfluidic channel (103). Further, the invention refers to a manufacturing method of such a sensing device (100).

## Description

The present invention refers to a method for manufacturing a sensing device for heavy metal detection in a liquid, in particular water, and such a sensing device.

On site water analysis as compared with laboratory analysis allows continuous monitoring of heavy metal concentration in water. This is important in order to reduce risks to humans from contaminated water and at the same time reduce the costs of performing laboratory analysis using expensive equipment and the associated labor costs. For that purpose, on-site measuring systems of heavy metals in water are under development including microfluidic devices and sensors. These systems must provide the optimum information to the user in terms of sensitivity, selectivity depending on the importance of each of these parameters for each application.

In particular, heavy metal ions, such as lead (Pb), cadmium (Cd), mercury (Hg) and chromium (Cr(III)), constitute one class of environmental pollutants with high chemical stability and low biodegradability. These metals accumulate in living organisms creating a wide range of negative effects such as cancer, kidney failure, diseases of the immune system and neurodegenerative diseases. Heavy metals are frequently detected in water wells, reservoirs, etc. and their impact in human health is severe as reported in epidemiological studies in the literature. Thus, there is an urgent need for development of portable devices to monitor the presence of heavy metal ions in potable water, in order to complement or replace the conventional detection methods currently used.

Conventional methods used today for the detection of heavy metal ions include spectroscopic techniques, (e.g., atomic absorption spectroscopy (AAS), inductively coupled plasma mass spectroscopy (ICP-MS), fluorescence spectroscopy), as well as other analytical methods such as high-performance liquid chromatography (HPLC). These methods are characterized by high accuracy and sensitivity but they usually require bulky and expensive equipment which are available in specially designed central laboratories and on top of that sample handling is usually time consuming and requires highly trained personnel.

Thus, a portable, low-cost, easy-to-fabricate and easy-to-operate device that can be used at the point of interest is of much interest and for that reason several paper-based detection solutions have been proposed in the literature. However, in these approaches detection is mainly done using colorimetry or other optical detection methods and there are applications (i.e., food and water safety) in which such devices cannot meet the requirements for low limit of detection (LOD) or quantification of results.

Therefore, there is an urgent need for the development of more portable detection systems using integrated sensors. To this end several examples of devices featuring sufficient LOD have been reported. For example, M. Zhang et al. have fabricated a digital microfluidic system with an electrochemical sensor for on-site detection of heavy metal ions in tap water. Rotake et al. have developed a portable microfluidic platform using a microcantilever based piezoresistive sensor for Cd(II) detection with a limit of detection (LOD) 2.78 pM, while Santangelo et al. combined an epitaxial graphene (EG) sensor with a 3D-printed microfluidic for heavy metal ion detection. They reported a quite low LOD of 95 nM for Pb2⁺, which they attributed to the high sensitivity of the sensing material. However, such approaches can be further improved by increasing the device integration degree and simplifying the device fabrication methods.

It is an object of the present invention to provide a sensing device for heavy metal detection in a liquid, in particular water, that is portable, low-cost, easy-to-fabricate and easy-to-operate while demonstrating high sensitivity, selectivity and flexibility to the user according to the needs of the application for which the sensing device is used, and a method for manufacturing such a sensing device.

In particular, a sensing device for heavy metal detection in a liquid, in particular water, is proposed that comprises a substrate, in particular oxidized silicon or a plastic substrate, a microfluidic channel, an MoS₂-based sensor (two-dimensional molybdenum disulfide sensor) and a nanoparticle network sensor (NP sensor), which is functionalized with DNAzymes. Both sensors, namely the MoS₂-based sensor and the nanoparticle network sensor, are integrated in the microfluidic channel. "Integrated" means in particular that the electrodes of the sensors are arranged inside the microfluidic channel so that they come in contact with the liquid that exists in or flows through the microfluidic channel. Due to the presence of the microfluidic channel, the sensing device can in particular also be characterized as a microfluidic sensing device. In the case where the substrate is an oxidized silicon substrate, the oxidized silicon substrate comprises a silicon substrate body covered by a silicone oxide layer. It is noted that the MoS₂-based sensor and nanoparticle network sensor are electrochemical sensors.

The described sensing device has the advantage of high sensitivity, selectivity and flexibility to the user according to the needs of the application for which the sensing device is used due to the two different material-based sensors with complementary roles for the detection of heavy metals in a liquid, in particular water, within the same microfluidic channel. In particular, due to its functionalization using DNAzymes, the nanoparticle network sensor is able to give selective information about metal concentration in water. Specifically, for each heavy metal to be detected a different type of DNAzyme is used. On the other hand, the MoS₂-based sensor can operate non-selectively at low heavy metal concentrations, in particular in the ppb range. It is understood that the DNAzymes, with which the nanoparticle-layer of the nanoparticle network sensor is functionalised, in the sensing device are of the same type. In other words, the nanoparticle network sensor in the sensing device is configured to detect one heavy metal, for which the used type of DNAzyme is specific. It is further noted that the term "heavy metal" is meant to be a heavy metal ion dissolved in the liquid.

To better understand the aforementioned advantages of the proposed sensing device, the contribution of the different components of the sensing device and their interplay to the sensing device are presented in more details in the following.

The use of DNAzymes can significantly enhance the selectivity of the sensing device. Further, when compared with other biomolecules, DNAzymes exhibit several advantages as they are more stable compared to RNA enzymes or proteins, their production cost is lower and they can also be denaturated and regenerated without affecting their catalytic ability or their ability to interact with the target analyte, in this case the heavy metals to be detected. These advantages make the proposed sensing device combining microfluidics technology and electrochemical sensing using DNA enzymes, in other words combining the microfluidic channel and the nanoparticle network sensor being functionalized with DNAzymes, a highly attractive solution, since the sensing device can also be made lightweight and portable for use in both laboratory and on-site measurements.

On the other hand, the use of the MoS₂-based sensor in the proposed sensing device is ideal for sensing applications of heavy metals, as the two-dimensional molybdenum disulfide (MoS₂) material, a member of metal dichalcogenides, has semiconducting behaviour, good optical and mechanical properties and high surface to volume ratio. This material can detect heavy metal traces when used as an electrochemical sensor however with lower selectivity than the nanoparticle network sensor. The detection mechanism is due to the high binding affinity between metals and the sulfur sites on the surface of the MoS₂ material.

In the propose sensing device, these two different sensor technologies are integrated into the same microfluidic channel for the selective detection of heavy metals in a cost-effective way: the non-selective MoS₂-based sensor that does not require any functionalization and the nanoparticle network sensor that is functionalized by DNAzymes in order to be able to selectively operate.

Having only nanoparticle network sensors in the sensing device would render the manufacturing process of the sensing device rather time-consuming, complex and expensive due to the need of a different functionalization (functionalisation) for detecting different heavy metals. The use of many selective sensors in a sensing system and particularly within the same microfluidic sensing device becomes then complicated and consequently might reduce the yield of the operating sensors. Instead, the combination of a MoS₂-based sensor and a nanoparticle network sensor (or more than two nanoparticle network sensors) gives an additional flexibility to the sensing device. For example, presence of contamination in water from a heavy metal that was not in a priority list of selected DNAzymes during the design of the sensing device can still be detected with the proposed sensing device. In that case, though the nanoparticle network sensor will not detect this heavy metal, as it is not functionalized with the DNAzyme specific for this heavy metal, the MoS₂-based sensor will still detect the presence of contamination in the water sample. This knowledge is of paramount importance to the user, even if the exact contaminant in the water is not known. In order to offer this level of flexibility that can be explored in different ways depending on the application case, the two different sensors are integrated within the same microfluidic channel.

Preferably, the MoS₂-based sensor and the nanoparticle network sensor are arranged one after the other in a longitudinal direction of the microfluidic channel. In other words, the MoS₂-based sensor and the nanoparticle network sensor are arranged in series. This means in particular that, when a liquid flows through the microfluidic channel, the liquid will first come in contact with one of the sensors and then with the other one. The longitudinal direction of the microfluidic channel may advantageously correspond to the direction of flow of a liquid through the microfluidic channel. The longitudinal direction of the microfluidic channel may preferably correspond to a longitudinal direction of the sensing device, in particular of the substrate.

Preferably, a thickness of a wall of the microfluidic channel is of hundred microns.

Preferably, the DNAzymes are specific for the detection of one of the following heavy metals: lead, cadmium, mercury and chromium (III). For example, the sensing device can be configured to detect lead in a liquid by means of the nanoparticle network sensor being functionalized with DNAzymes of the type that is specific for the detection of lead and further configured to generally detect the presence of a heavy metal in the liquid and thus the contamination of the liquid. For example, if the liquid contains cadmium, this will be detected by means of the MoS₂-based sensor in such a sensing device.

Advantageously, the MoS₂-based sensor comprises a two-dimensional MoS₂ layer and a pair of electrodes.

According to an advantageous embodiment, the sensing device may comprise one (only one) MoS₂-based sensor and at least one nanoparticle network sensor, in particular a plurality of nanoparticle network sensor each configured to detect a different heavy metal.

Further proposed is a method for manufacturing a sensing device for heavy metal detection in a liquid, in particular water, the sensing device comprising a substrate, in particular an oxidized silicon or a plastic substrate, a microfluidic channel, an MoS₂-based sensor (two-dimensional molybdenum disulfide sensor) and a nanoparticle network sensor (NP sensor), which is functionalized with DNAzymes, wherein both sensors, namely the MoS₂-based sensor and the nanoparticle network sensor, are integrated in the microfluidic channel. In particular, the method is for manufacturing the previously described sensing device.

The method comprises the steps of providing a substrate, forming a MoS₂-based sensor on the substrate, forming a nanoparticle network sensor on the substrate, functionalizing the nanoparticle network sensor with DNAzymes, and fabricating a microfluidic channel on the substrate. The microfluidic channel, the MoS₂-based sensor and the nanoparticle sensor are formed such that the MoS₂-based sensor and the nanoparticle network sensor are integrated in the microfluidic channel. In other words, the step of forming the microfluidic channel, the step of forming the MoS₂-based sensor and the step of forming the nanoparticle sensor are done such that the MoS₂-based sensor and the nanoparticle network sensor are integrated in the microfluidic channel.

It is noted that these steps, in particular the steps of forming a MoS₂-based sensor on the substrate, forming a nanoparticle network sensor on the substrate, functionalizing the nanoparticle network sensor with DNAzymes, and fabricating a microfluidic channel on the substrate, may or may not be executed in the sequence/order presented above. For example, the fabrication of the microfluidic channel may be executed before the formation of the nanoparticle network sensor. Further, the aforementioned steps, in particular the steps of forming a nanoparticle network sensor on the substrate, functionalizing the nanoparticle network sensor with DNAzymes, and fabricating a microfluidic channel on the substrate, may be partially overlapping. This means that a sub-step of one of the aforementioned steps may be executed together with a sub-step of another one of the described steps, in particular in a single process. Preferably, the MoS₂-based sensor is formed prior to the steps of forming the nanoparticle network sensor on the substrate, its functionalization with DNAzymes and the fabrication of the microfluidic channel on the substrate. This means that all the sub-steps of the step of forming the MoS₂-based sensor on the substrate are preferably executed before any of the sub-steps of the steps of forming the nanoparticle network sensor, the functionalizing step and the fabrication step as presented above.

According to an advantageous embodiment, the formation of the MoS₂-based sensor is executed first, then the fabrication of the microfluidic channel, and after that the formation of the nanoparticle network sensor and its functionalization with DNAzymes. It is understood that the execution of the step of forming the MoS₂-based sensor results in the MoS₂-based sensor, the execution of the step of fabricating the microfluidic channel in the microfluidic channel and the execution of the step of forming the nanoparticle network sensor in the nanoparticle sensor. According to an advantageous configuration, between the step of forming the MoS₂-based sensor and the step of fabricating the microfluidic channel, the formation of the electrodes of the nanoparticle network sensor can be executed, resulting in the electrodes of the nanoparticle network sensor. The remaining steps for forming the nanoparticle network sensor can be executed after the execution of the step of fabricating the microfluidic channel. According to an alternative configuration, all the steps of forming the nanoparticle network sensor are executed after the fabrication of the microfluidic channel.

The substrate may in particular be an oxidized silicon substrate or a plastic substrate.

Preferably, the step of forming the MoS₂-based sensor comprises a sub-step of forming a layer, in particular monolayer, of MoS₂ on the substrate. In particular, the layer of MoS₂ can be formed on the substrate being an oxidized silicon substrate, or on a starting substrate being an oxidized silicon substrate and then transferred on a plastic substrate. The plastic substrate corresponds here to the previously mentioned substrate (not to be confused with the starting substrate).

Preferably, the step of forming the MoS₂-based sensor further comprises a sub-step of depositing a lithographic resist over a predetermined region (predetermined surface area) of the layer of MoS₂. The predetermined region is smaller than the complete layer of MoS₂, thereby forming a first region of the layer of MoS₂ being protected by the lithographic resist and a second region of the layer of MoS₂ without lithographic resist. The predetermined region particularly corresponds to the region that the MoS₂-based sensor will be arranged in the sensing device.

Preferably, the step of forming the MoS₂-based sensor further comprises a sub-step of removing the second region of the MoS₂ layer. This can in particular be done by using oxygen plasma.

Preferably, the step of forming the MoS₂-based sensor further comprises a sub-step of removing the lithographic resist. This can in particular be done by wet cleaning of the lithographic resist with acetone followed by wet cleaning with propanol. However, it is possible to use another resist remover and/or solvent.

Preferably, the step of forming the MoS₂-based sensor further comprises a sub-step of fabricating a pair of electrodes, in particular by a lithography lift-off process, for example an optical or electron beam lithography lift-off process. These electrodes can also be referred to as first electrodes.

Instead of using a lithography lift-off process, it is also possible that the fabrication of the electrodes of the MoS₂-based sensor can be done by first depositing a metal film on the MoS₂ layer, then use lithography to define the electrode pattern on a resist film and then etch away the unprotected by the resist metal. However, the lift-off lithography process is preferable compared to said alternative process, as with the alternative process there might exist a risk that the underlying MoS₂ layer is etched away too during metal etching, if not properly executed.

Preferably, the step of forming the nanoparticle network sensor comprises fabricating a pair of electrodes, in particular by lithography lift-off process.

The electrodes of the nanoparticle network sensor and the electrodes of the MoS₂-based sensor are fabricated in a single process.

Preferably, the step of fabricating the microfluidic channel comprises depositing a lithographic resist over the MoS₂-based sensor and depositing an oxide layer at a temperature lower than 100 degrees Celsius over the lithographic resist. The oxide layer may in particular be a silicon oxide layer or a silicon nitride layer. In particular, the deposition may be done by sputtering or atomic layer deposition. The oxide layer guarantees no attack of the lithographic resist deposited over the MoS₂-based sensor during subsequent formation of the microfluidic channel. The oxide layer preferably has a thickness between 10 nm and 100 nm.

Said lithographic resist is preferably of submicron thickness.

Preferably, the step of fabricating the microfluidic channel further comprises applying a dry resist film on the oxide layer and patterning the dry resist film for forming the microfluidic channel. Preferably, the substrate with the applied dry resist film is passed through a laminator before patterning it, in particular wherein a temperature of the laminator is set between 100 degrees Celsius and 150 degrees Celsius. Thus, the adhesion of the dry resist film can be omptimized. Lithographic patterning can preferably be used for patterning the dry resist. This guarantees batch fabrication (mass production) of the sensing device. Alternatively, a polydimethylsiloxane material may be mould and applied over the MoS₂-based sensor and the nanoparticle network sensor for forming the microfluidic channel. The polydimethylsiloxane material may also be patterned using lithographic techniques that makes it more appropriate for mass production of the sensing device.

It is noted that attention must be paid not to change protective lithographic resist properties during processing of dry resist. Advantageously, in order to make sure that this is the case, the processing temperature is lower than 120 degrees Celsius.

The application of the polydimethylsiloxane material over the MoS₂-based sensor and the nanoparticle network sensor implies that the application step takes place after the MoS₂-based sensor and the nanoparticle network sensor are formed.

Preferably, the step of forming the nanoparticle network sensor further comprises removing the deposited oxide layer, and depositing nanoparticles, in particular metallic nanoparticles, on the electrodes of the nanoparticle network sensor. The removal of the deposited oxide layer may particularly be done with hydrofluoric acid solution or by dry etching using fluorine chemistry plasma (CHF3 or SF6). The removal of the deposited oxide layer occurs after the step of fabricating the microfluidic channel, in particular after patterning the dry resist film.

According to an advantageous embodiment, the step of depositing nanoparticles comprises depositing nanoparticles by a gas condensation process in vacuum. In that case, nanoparticles are preferably from gold or platinum to avoid their oxidation and can in particular be deposited using a sputtering-based technique or laser-assisted ablation of a metal target.

According to an alternative advantageous embodiment, the step of depositing nanoparticles comprises depositing nanoparticles by ink-jet printing, spin coating or drop casting nanoparticles contained in a solution. This advantageously takes place in ambient. In particular, the nanoparticles are prepared by chemical methods and kept within a solution, which is then ink-jet printed or drop casted on the electrodes of the nanoparticle network sensor.

Preferably, the step of functionalizing the nanoparticle network sensor with DNAzymes comprises a sub-step of removing the lithographic resist deposited over the MoS₂-based sensor, and a sub-step of depositing DNAzymes selectively on the nanoparticle network sensor, in particular by using automatic equipment or by manual drop casting. The removal of the lithographic resist deposited over the MoS₂-based sensor guarantees that DNAzymes will not be deposited on the MoS₂-based sensor, namely the two-dimensional MoS₂ material and its electrodes, but only to the electrodes formed for the nanoparticle network sensor. The removal of the lithographic resist deposited over the MoS₂-based sensor may in particular be done by wet cleaning of the lithographic resist with acetone followed by wet cleaning with propanol. In particular, oxygen plasma should be avoided for removing the lithographic resist as there is the risk of etching the MoS₂ layer.

According to an advantageous embodiment, the DNAzymes may be thiolated DNAzymes. According to an alternative advantageous embodiment, the DNAzymes may be amino-terminated. In the latter case, the nanoparticle network sensor may in particular be functionalised before depositing the DNAzymes using 3-aminopropyltriethoxysilane deposition (APTES deposition).

These and further details, advantages and features of the present invention will be described based on an embodiment of the invention by taking reference to the accompanying figures.
Figure 1a is a schematic simplified representation of a first sub-step of a method for manufacturing a sensing device for heavy metal detection according to an embodiment of the present invention.
Figure 1b is another schematic simplified representation of the first sub-step of the method according to the embodiment of the present invention.
Figure 2a is a schematic simplified representation of a second sub-step of a method for manufacturing a sensing device for heavy metal detection according to an embodiment of the present invention.
Figure 2b is another schematic simplified representation of the second sub-step of the method according to the embodiment of the present invention.
Figure 3a is a schematic simplified representation of a third sub-step of a method for manufacturing a sensing device for heavy metal detection according to an embodiment of the present invention.
Figure 3b is another schematic simplified representation of the third sub-step of the method according to the embodiment of the present invention.
Figure 4a is a schematic simplified representation of a fourth sub-step of a method for manufacturing a sensing device for heavy metal detection according to an embodiment of the present invention.
Figure 4b is another schematic simplified representation of the fourth sub-step of the method according to the embodiment of the present invention.
Figure 5a is a schematic simplified representation of a fifth sub-step of a method for manufacturing a sensing device for heavy metal detection according to an embodiment of the present invention.
Figure 5b is another schematic simplified representation of the fifth sub-step of the method according to the embodiment of the present invention.
Figure 6a is a schematic simplified representation of a sixth sub-step of a method for manufacturing a sensing device for heavy metal detection according to an embodiment of the present invention.
Figure 6b is another schematic simplified representation of the sixth sub-step of the method according to the embodiment of the present invention.
Figure 7a is a schematic simplified representation of a seventh sub-step of a method for manufacturing a sensing device for heavy metal detection according to an embodiment of the present invention.
Figure 7b is another schematic simplified representation of the seventh sub-step of the method according to the embodiment of the present invention.
Figure 8a is a schematic simplified representation of an eighth sub-step of a method for manufacturing a sensing device for heavy metal detection according to an embodiment of the present invention.
Figure 8b is another schematic simplified representation of the eighth sub-step of the method according to the embodiment of the present invention.
Figure 9a is a schematic simplified representation of a ninth sub-step of a method for manufacturing a sensing device for heavy metal detection according to an embodiment of the present invention.
Figure 9b is another schematic simplified representation of the ninth sub-step of the method according to the embodiment of the present invention.
Figure 10a is a schematic simplified representation of a tenth sub-step of a method for manufacturing a sensing device for heavy metal detection according to an embodiment of the present invention.
Figure 10b is another schematic simplified representation of the tenth sub-step of the method according to the embodiment of the present invention.

In the following, a method for manufacturing a sensing device for heavy metal detection in a liquid, in particular water, and a sensing device according to an embodiment of the present invention are described in detail by taking reference to accompanying figures.

The method comprises the steps of providing a substrate, forming a MoS₂-based sensor on the substrate, forming a nanoparticle network sensor on the substrate, functionalizing the nanoparticle network sensor with DNAzymes, and fabricating a microfluidic channel on the substrate. The microfluidic channel, the MoS₂-based sensor and the nanoparticle sensor are formed such that the MoS₂-based sensor and the nanoparticle network sensor are integrated in the microfluidic channel.

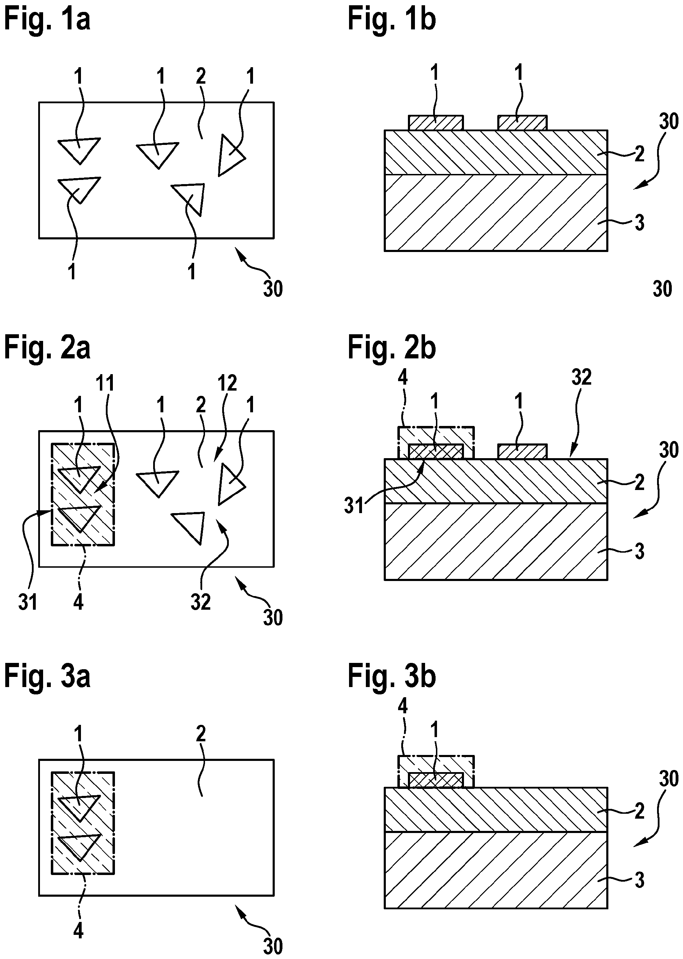

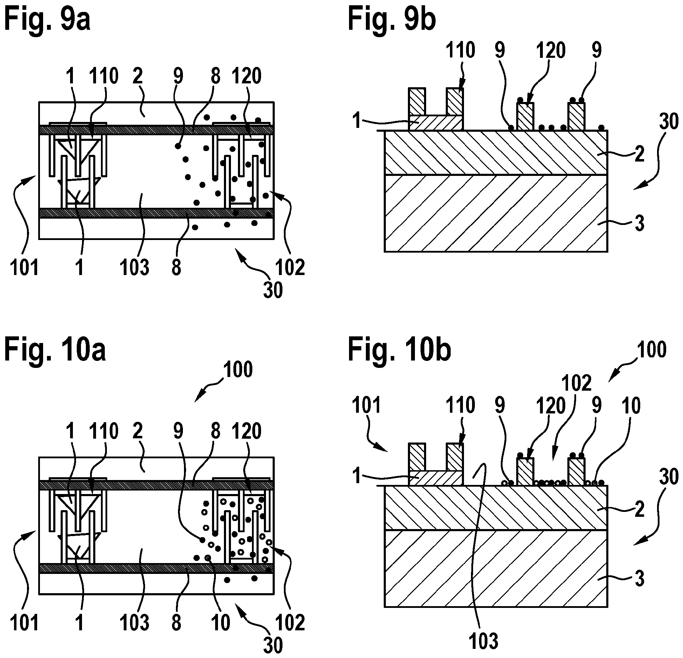

These steps will be explained in detail with reference to figures 1a,b to 10a,b. Figures 1a to 10a are each a top view of the substrate, while figures 1b to 10b are each a side view of the substrate.

In a first sub-step of the method, the result of which is shown in figures 1a and 1b, a layer 1 of MoS₂ is formed on a substrate 30. In the present embodiment, the substrate 30 is in particular an oxidized silicon substrate that comprises a substrate body 3 and an oxide layer 2. In particular, the layer 1 of MoS₂ is formed on the oxide layer 2. Alternatively, the layer 1 of MoS₂ can be formed on a starting substrate being an oxidized silicon substrate like the one shown in figures 1a and 1b and then transferred on a plastic substrate. In this case, the substrate 30 of the present invention is the plastic substrate on which the layer 1 of of MoS₂ is transferred and the rest of the method is executed with the plastic substrate.

The layer 1 of of MoS₂ is shown in the figures as non-continuous layer and represented as triangles. The shape is exemplary for illustration purposes only. It is understood that the layer 1 of MoS₂ can be formed as a continuous layer. The layer 1 of MoS₂ may be formed by depositing MoS₂ on the substrate 30, for example by sputtering.

In a second sub-step of the method, the result of which is shown in figures 2a and 2b, a lithographic resist 4 (first lithographic resist) is deposited over a predetermined region of the layer 1 of MoS₂. The predetermined region is smaller than the complete region of the layer 1 of MoS₂, thereby forming a first region 11 of the layer 1 of MoS₂ being protected by the lithographic resist and a second region 12 of the layer 1 of MoS₂ without lithographic resist. As can be seen from figures 1a and 1b, the layer 1 of MoS₂ in the predetermined region, i.e., the first region 11 of the layer 1, is completely covered by the lithographic resist 4. It is thus understood that a first substrate region 31 of the substrate 30 is (directly and indirectly due to the presence of the first region of the layer 1 of MoS₂) covered by the lithographic resist 4, while a second substrate region 32 of the substrate 30 is free of lithographic resist. In other words, the lithographic resist 4 is covers the first region 1 of the layer 1 as well as part of the substrate surface, in particular the oxide layer surface.

In a third sub-step of the method, the result of which is shown in figures 3a and 3b, the second region 12 of the layer 1 of MoS₂ is removed. This can in particular be done by using oxygen plasma.

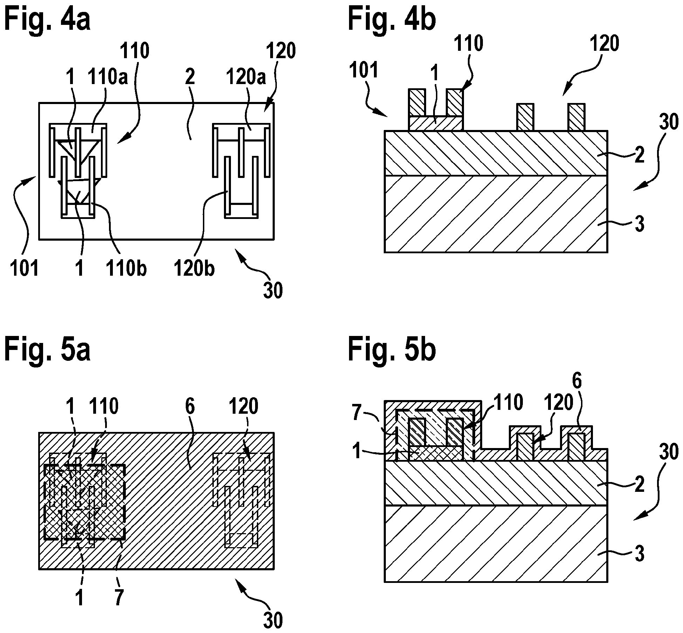

Figures 4a and 4b refer to a fourth sub-step of the method.

In the fourth sub-step of the method, the lithographic resist 4 is removed. This can in particular be done by wet cleaning of the lithographic resist 4 with acetone followed by wet cleaning with propanol.

Further, in the fourth sub-step of the method, a pair of electrodes 110, in particular an anode 110a and a cathode 110b, are fabricated for the MoS₂-based sensor. This can in particular be done by a lithography lift-off process, for example an optical or electron beam lithography lift-off process. The electrodes 110 for the MoS₂-based sensor can also be referred to as first electrodes.

The result of the first, second, third and fourth sub-steps is the formation of a MoS₂-based sensor 101. In other words, the first, second, third and fourth sub-steps constitute the step of forming the MoS₂-based sensor 101.

As can be seen from figures 4a, 4b, in the fourth sub-step of the method, a further pair of electrodes 120, in particular a further anode 120a and a further cathode 120b, are fabricated. The electrodes 120 are intended to be the electrodes of the nanoparticle network sensor to be formed. These electrodes 120 can be also referred to as second electrodes. The first electrodes 110 and the second electrodes 120 are fabricated in a single process (step), in particular in a single lithography lift-off process. Thus, the fourth sub-step of the method is also part of the step of forming the nanoparticle network sensor.

Figures 5a and 5b show the result of a sixth sub-step of the method.

In the sixth sub-step of the method, a lithographic resist 7 (second lithographic resist) is deposited over the MoS₂-based sensor 101. In addition, an oxide layer 6 is deposited at a temperature lower than 100 degrees Celsius over the lithographic resist 7. The oxide layer 6 may in particular be a silicon oxide layer or a silicon nitride layer. In particular, the deposition of the oxide layer 6 may be done by sputtering or atomic layer deposition. The oxide layer 6 ensures that the lithographic resist 7 deposited over the MoS₂-based sensor 101 is protected during subsequent formation of the microfluidic channel. The oxide layer 6 preferably has a thickness between 10 nm and 100 nm.

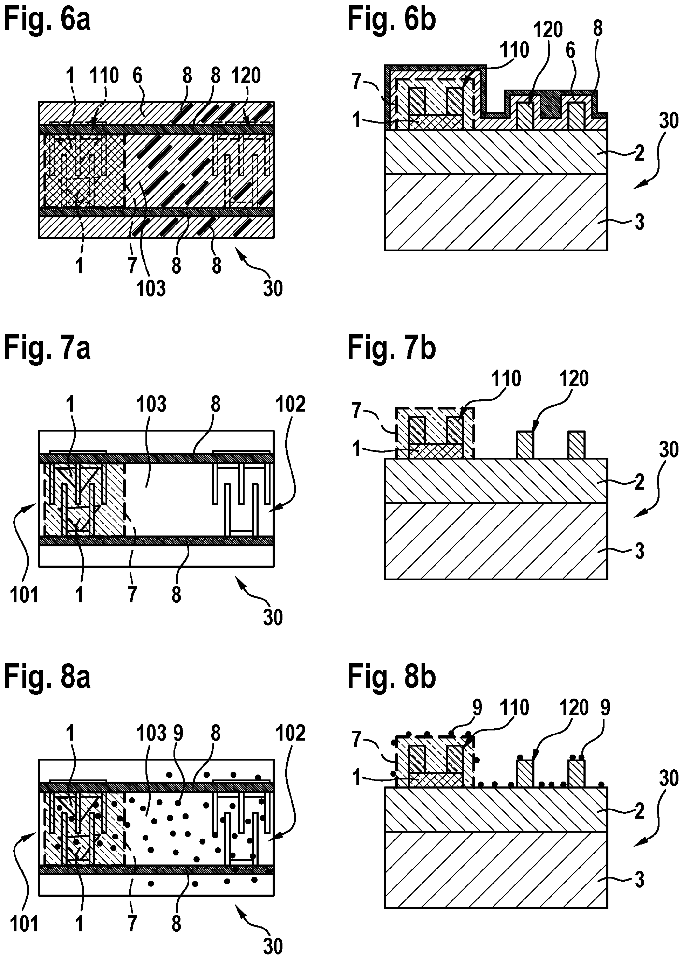

With reference to figures 6a and 6b, in a seventh sub-step, a dry resist film 8 is applied on the oxide layer 6 and patterned for forming (shaping) a microfluidic channel 103. Here, the substrate 30 with the applied dry resist film 8 is passed through a laminator before patterning the dry resist film 8. This has the advantage of optimizing the adhesion of the dry resist film 8 on the substrate 30, in particular on the oxide layer 6. In particular, the temperature of the laminator is set between 100 degrees Celsius and 150 degrees Celsius.

The result of the sixth and the seventh sub-steps is the fabrication of the microfluidic sensor 103.

In an eighth sub-step, the result of which is shown in figures 7a and 7b, the deposited oxide layer 6 is (completely) removed. The removal of the deposited oxide layer 6 may particularly be done with hydrofluoric acid solution or by dry etching using fluorine chemistry plasma (CHF3 or SF6). The removal of the deposited oxide layer 6 occurs after the step of fabricating the microfluidic channel, in particular after patterning the dry resist film 8. It is noted that the final form of the microfluidic channel 103 is shown in figures 7a and 7b. The microfluidic channel 103 is formed such that the electrodes 110 and the electrodes 120 are inside the microfluidic channel 103. It is noted that the electrodes 110, 120 of the sensors partially extend out of the microfluidic channel 103 such that an electrical measurement can take place. The electrodes 110, 120 are configured to measure either the electrical resistance under constant voltage or the impedance under alternating voltage.

In a nineth sub-step, the result of which is shown in figures 8a and 8b, metallic nanoparticles 9 are deposited on the electrodes 120 intended for the nanoparticle network sensor. This can in particular be done by a gas condensation process in vacuum. The nanoparticles 9 are preferably from gold or platinum to avoid their oxidation and can in particular be deposited using a sputtering-based technique or laser-assisted ablation of a metal target.

Alternatively, the nanoparticles 9 can be deposited by ink-jet printing, spin coating or drop casting nanoparticles contained in a solution. This advantageously takes place in ambient. In particular, the nanoparticles 9 are prepared by chemical methods and kept within a solution, which is then ink-jet printed or drop casted on the electrodes 120 of the nanoparticle network sensor.

The fifth, eighth and nineth sub-steps constitute the step of forming a nanoparticle network sensor 102. In other words, the result of said sub-steps id the formation of the nanoparticle network sensor 103.

In a tenth sub-step, the result of which is shown in figures 9a and 9b, the lithographic resist 7 deposited over the MoS₂-based sensor 101 is removed.

In an eleventh step, the result of which is shown in figures 10a and 10b, DNAzymes 10 are selectively deposited on the nanoparticle network sensor 102. This can be done by using automatic equipment or by manual drop casting. The removal of the lithographic resist 7 deposited over the MoS₂-based sensor 101 guarantees that DNAzymes will not be deposited on the MoS₂-based sensor 101, namely the two-dimensional MoS₂ material and its electrodes 110, but only to the electrodes 120 of nanoparticle network sensor 102. The removal of the lithographic resist 7 deposited over the MoS₂-based sensor 101 may in particular be done by wet cleaning of the lithographic resist 7 with acetone followed by wet cleaning with propanol.

The DNAzymes 10 may be thiolated DNAzymes. Alternatively, the DNAzymes 10 may be amino-terminated. In the case of amino-terminated DNAzymes 10, the nanoparticle network sensor 102 may in particular be functionalised before depositing the DNAzymes 10 using 3-aminopropyltriethoxysilane deposition (APTES deposition).

The tenth and eleventh sub-steps constitute the step of functionalising the nanoparticle network sensor 102.

The manufactured sensing device 100 is shown in figures 10a and 10b.

By the present invention, a compact, low-cost, easy-to-fabricate, easy-to-operate and portable sensing device 100 that also demonstrates high sensitivity, selectivity and flexibility to the user according to the needs of the application for which the sensing device 100 is used.

### Reference signs

- 1: layer of MoS₂ (MoS₂ layer)
- 2: oxide layer
- 3: substrate body
- 4: lithographic resist (first lithographic resist)
- 6: oxide layer
- 7: lithographic resist (second lithographic resist)
- 8: dry resist film

- 10: DNAzymes
- 11: first region
- 12: second region
- 30: substrate
- 31: first substrate region
- 32: second substrate region

- 100: sensing device
- 101: MoS₂-based sensor
- 102: nanoparticle network sensor
- 103: microfluidic channel
- 110: electrodes (first electrodes)
- 110a: anode (first anode)
- 110b: cathode (first cathode)
- 120: electrodes (second electrodes)
- 120a: anode (second anode)
- 120b: cathode (second cathode)

## Claims

1. A sensing device (100) for heavy metal detection in a liquid, in particular water, comprising:
• a substrate (30),
• a microfluidic channel (103),
• an MoS₂-based sensor (101), and
• a nanoparticle network sensor (102), which is functionalized with DNAzymes (10), wherein the MoS₂-based sensor (101) and the nanoparticle network sensor (102) are integrated in the microfluidic channel (103).

2. The sensing device (100) according to claim 1, wherein the substrate (30) is an oxidized silicon substrate.

3. The sensing device (100) according to claim 1, wherein the substrate (30) is a plastic substrate.

4. The sensing device (100) according to any of the preceding claims, wherein the MoS₂-based sensor (101)and the nanoparticle network sensor (102) are arranged one after the other in a longitudinal direction of the microfluidic channel (103).

5. The sensing device (100) according to any of the preceding claims, wherein the DNAzymes (10) are specific for the detection of one of lead, cadmium, mercury and chromium (III).

6. A method for manufacturing a sensing device (100) for heavy metal detection in a liquid, in particular water, in particular for manufacturing the sensing device (100) according to any of the preceding claims, comprising the steps:
• providing a substrate (30), in particular an oxidized silicon substrate or a plastic substrate,
• forming a MoS₂-based sensor (101)on the substrate (30),
• forming a nanoparticle network sensor (102) on the substrate (30),
• functionalizing the nanoparticle network sensor (102) with DNAzymes, and
• fabricating a microfluidic channel (103) on the substrate (30),
wherein the microfluidic channel (103), the MoS₂-based sensor (101) and the nanoparticle sensor (101) are formed such that the MoS₂-based sensor (101) and the nanoparticle network sensor (102) are integrated in the microfluidic channel (103).

7. The method of claim 6, wherein the step of forming the MoS₂-based sensor comprises the sub-steps:
• forming a layer (1), in particular monolayer, of MoS₂ on the substrate (30),
• depositing a lithographic resist (4) over a predetermined region of the layer (1) of MoS₂, wherein the predetermined region is smaller than the complete layer (1) of MoS₂, thereby forming a first region (11) of the layer (1) of MoS₂ being protected by the lithographic resist and a second region (12) of the layer (1) of MoS₂ without lithographic resist,
• removing the second region (12) of the layer (1) of MoS₂ , in particular by using oxygen plasma,
• removing the lithographic resist (4), in particular by wet cleaning of the lithographic resist with acetone followed by wet cleaning with propanol,
• and fabricating a pair of electrodes (110), in particular by a lithography lift-off process.

8. The method of claim 7, wherein forming the layer (1) of MoS₂ comprises depositing a layer of MoS₂ on the substrate (30) being an oxidized silicon substrate.

9. The method of claim 7, wherein forming the layer (1) of MoS₂ comprises depositing a layer of MoS₂ on a starting substrate being an oxidized silicon substrate and transferring said layer on the substrate (30) being a plastic substrate.

10. The method of any of claims 7 to 9, wherein the step of forming the nanoparticle network sensor (102) comprises fabricating a pair of electrodes (120), in particular by lithography lift-off process, wherein the electrodes (120) of the nanoparticle network sensor (102) and the electrodes (110) of the MoS₂-based sensor (101) are fabricated in a single process.

11. The method of any of claims 6 to 10, wherein the step of fabricating the microfluidic channel (103) comprises:
• depositing a lithographic resist (7) over the MoS₂-based sensor (101),
• depositing an oxide layer (6), in particular a silicon oxide layer or a silicon nitride layer, at a temperature lower than 100 degrees Celsius over the lithographic resist (7), in particular by sputtering or atomic layer deposition, and
• applying a dry resist film (8) on the oxide layer (6) and patterning the dry resist film (8) for forming the microfluidic channel (103), preferably wherein the substrate (30) is passed through a laminator before patterning it, in particular wherein a temperature of the laminator is set between 100 degrees Celsius and 150 degrees Celsius, or
molding a polydimethylsiloxane material and applying it over the MoS₂-based sensor (101) and the nanoparticle network sensor (102) for forming the microfluidic channel (103).

12. The method of claim 11, wherein the step of forming the nanoparticle network sensor (103) further comprises the sub-steps:
• removing the deposited oxide layer (6), in particular with hydrofluoric acid solution or by dry etching using fluorine chemistry plasma, and,
• depositing nanoparticles (9) on the electrodes (120) of the nanoparticle network sensor (102).

13. The method of claim 12, wherein the sub-step of depositing nanoparticles (9) comprises depositing nanoparticles (9) by a gas condensation process in vacuum.

14. The method of claim 12, wherein the sub-step of depositing nanoparticles (9) comprises depositing nanoparticles (9) by ink-jet printing, spin coating or drop casting nanoparticles contained in a solution.

15. The method any of claims 11 to 14, wherein the step of functionalizing the nanoparticle network sensor (102) with DNAzymes (10) comprises:
• removing the lithographic resist (7) deposited over the MoS₂-based sensor (101), in particular by wet cleaning of the lithographic resist (7) with acetone followed by wet cleaning with propanol,
and
• depositing DNAzymes (10) selectively on the nanoparticle network sensor (102),
in particular:
wherein the DNAzymes (10) are thiolated DNAzymes, or
wherein the DNAzymes (10) are amino-terminated, in particular wherein the nanoparticle network sensor (102) is prepared before depositing the DNAzymes (10) using 3-aminopropyltriethoxysilane deposition.
